# EUROPEAN PATENT SPECIFICATION

(11) **EP 1 767 167 B1**
(45) Date of publication and mention of the grant of the patent: **18.05.2011**
(21) Application number: 06121084.5
(22) Date of filing: 22.09.2006
(51) Int. Cl.: A61B 19/00, A61B 10/02

(54) **Post decompression marker introducer system**
System zum Einsetzen eines Markers nach Druckentlastung
Système pour l'introduction d'un marqueur après décompression

(30) Priority: 26.09.2005 US 596467
(43) Date of publication of application: 28.03.2007
(73) Proprietor: Bard Peripheral Vascular, Inc., Tempe, AZ 85280-1740 (US); Bard Shannon Limited, 3431 HR Nieuwegein (NL)
(72) Inventor: Field, Steven E., Grand Rapids, MI 49546 (US)
(74) Representative: Tomlinson, Edward James

(56) References cited:
- US-B1- 6 234 177
- US-B2- 6 712 774

## Description

### Field of the Invention

This invention relates generally to a medical device for marking a target site within a tissue mass and more specifically to a medical device having a marker for marking a biopsy site in breast tissue that is deployed after the breast tissue has been decompressed.

### Description of the Related Art

A biopsy is a well-known medical procedure that involves taking a sample of tissue from a person and examining it for diagnostic purposes. This is often done when an abnormality is found in a tissue mass, for example when a lump is found in breast tissue or when an imaging system, such as mammography or ultrasonography detects a suspicious area. Examining a sample of tissue from an abnormal site or lesion is currently the only way to accurately diagnose cancer.

A vacuum-assisted biopsy (VAB) uses an imaging system, such as ultrasonography or mammography, to locate a lesion in the breast tissue and to guide a biopsy probe to the site. An example of a known VAB device 200 is shown in Fig. 1. Such a VAB device is described in United States Patent No. 6,712,774. The details of the VAB device are not germane to the invention and thus will only be briefly described. The probe 70 has a pointed tip 76 to facilitate its insertion through the tissue mass, an opening 78 in the side wall of the probe near the pointed tip, and a vacuum chamber 72. Once the probe 70 is in position at the lesion site, a vacuum pump creates a vacuum in chamber 72 and draws the tissue through the opening 78 and into a sampling chamber where a cutting device is advanced through the probe 70 to cut and remove a tissue sample. Other instruments can be inserted through the probe 70 in addition to the cutting device.

The position of the patient during VAB depends on the imaging system used to locate the lesion and position the probe. If ultrasonography is used, the patient will be in a supine position. If mammography is used, the patient typically lies prone on a specialized table such that the breast protrudes through a hole in the table. The breast is compressed between two plates while an image of the lesion is produced on a monitor by a mammography unit. Once the lesion is imaged, the VAB probe, which is mounted to the table or the mammography unit, is inserted into the breast tissue and the tissue sample is gathered as described above.

In some cases, it is desirable to mark the location of the lesion site in case a future biopsy or surgery is necessary. This is done with a marker that is made of any suitable material that can be imaged by an imaging system, such as ultrasonography, magnetic resonance, or mammography, or that is palpable through the skin and tissue of the patient. The marker must be accurately placed at the lesion site in the breast tissue and must remain at the site so that the lesion can be located and identified at a later time, if necessary. However, there sometimes is a need for a marker to be repositioned after its initial placement, such as if the marker was not placed at the desired location or if the marker shifts upon decompression of the tissue. Thus, the marker must be able to remain anchored in the breast tissue, yet permit its repositioning.

One type of marker is a biocompatible clip that can be placed at the lesion site to facilitate locating the lesion during later procedures. The clip has the advantage of being implanted entirely within the tissue mass, so that there is no possibility of accidental repositioning by pulling or tugging the clip. The clip is placed after the tissue sample has been gathered from the lesion site and while the breast is still compressed. The clip is inserted into the tissue mass through the VAB probe and thus does not require the tissue mass to be repierced. Since the clip is deployed when the breast tissue is compressed, upon decompression the clip may be found to be implanted away from the lesion site, leading to inaccurate marking of the lesion site. An illustrative example of the post-decompression shifting problem is a rubber ball that is normally 5 cm in diameter, but compressed to 2 cm. If a clip is to be placed 1 cm from the edge of the ball, the clip would be placed at the center of the ball. However, if upon decompression of the ball the clip stays at the center of the ball or shifts away from the target site, the clip is misplaced by up to several centimeters. Coopers ligaments in the breast exacerbate the problem of inaccurate marking by acting to pull the clip away from the site of implantation when the breast is uncompressed.

US 6,234,177 describes an apparatus for deploying an expandable biopsy marker. In the apparatus, a deployment catheter having an axial catheter lumen is provided, with the marker being deployed by a plunger axially received in the lumen of the catheter. The preamble of claim 1 is based on this prior art.

### SUMMARY OF THE INVENTION

According to the present invention, an apparatus for implanting a locatable marker at a target site within a tissue mass comprises a insertion device comprising a lumen having an exit opening, an introducer system comprising a sheath slidably received within the lumen and comprising a first lumen having a distal opening, a locatable marker received within the first lumen and deployable through the distal opening, and an anchor operably coupled to the sheath to fix the location of the sheath in the tissue mass, wherein the insertion device can be located within the tissue mass and the sheath can be inserted into the tissue mass through the exit opening of the insertion device, and the anchor can fix the position of the sheath in the tissue mass for deployment of the locatable marker at the target site.

The introducer system can comprise a second lumen having a distal opening, with the anchor received within the second lumen and deployable through the distal opening. The introducer system can comprise a distal terminal end and the distal terminal end can comprise an insertion tip. At least one of the introducer system distal openings can be formed in the terminal end of the introducer system. At least one of the introducer system distal openings can be formed in a side wall of the introducer system. At least one of the sheath distal openings formed in the side wall can comprise a ramp to guide the locatable marker through the at least one of the introducer system distal openings formed in the side wall.

The apparatus can further comprise a pushrod slidably received within the first lumen that deploys the locatable marker through the distal opening.

The anchor can comprise an anchor wire. The anchor wire can be operable between a straight configuration where the anchor wire is contained within the second lumen and a curved configuration where the anchor wire is extended through a distal opening. The anchor wire can be embedded in the tissue mass in the curved configuration.

The apparatus can further comprise a cannula received within the insertion device lumen, the cannula comprising a cannula lumen having a distal opening, with the introducer system received within the cannula lumen. The cannula distal opening can comprise a ramp to guide the introducer system through the cannula distal opening.

The insertion device can be a biopsy probe. The probe can be a vacuum-assisted biopsy probe. The exit opening can comprise a ramp. The introducer system can be flexible. The introducer system can comprise distance markings.

The apparatus can further comprising a pair of compression plates for compressing the tissue mass prior to location of the insertion device into the tissue mass at the target site and for decompressing the tissue mass prior to implantation of the locatable marker.

The apparatus can further comprise a hemostatic agent received within the first lumen and deployable through the distal opening. The locatable marker can be one of an imaging marker and a palpable marker. The locatable marker can be a clip.

### BRIEF DESCRIPTION OF THE DRAWINGS

In the drawings:
Fig. 1 is a schematic illustration of a prior art VAB device.
Fig. 2 is a schematic illustration of a VAB probe containing an introducer system according to the first embodiment of the present invention comprising a sheath having a first lumen containing a marker clip and a pushrod and a second lumen containing an anchor wire.
Fig. 3 is an enlarged view of the first embodiment of the introducer system from Fig. 2.
Fig. 4 is a sectional view of the introducer system taken along line 4-4 from Fig. 3.
Fig. 5 is a drawing similar to Fig. 3 illustrating the anchor wire extended from the second lumen.
Fig. 6 is a drawing similar to Fig. 5 illustrating the pushrod extended from the first lumen to push the marker clip out of the introducer system.
Fig. 7 is a schematic illustration of the VAB probe from Fig. 2 inserted into a tissue mass comprising a breast that is compressed between compression plates.
Fig. 8 is a close-up view of area VIII from Fig. 7 illustrating the insertion of the introducer system into the VAB probe.
Fig. 9 is a drawing similar to Fig. 8 illustrating the introducer system extended from the VAB probe and into the tissue mass.
Fig. 10 is a drawing similar to Fig. 9 illustrating the anchor wire extended from the second lumen and anchored in the tissue mass.
Fig. 11 is a drawing illustrating the tissue mass in an uncompressed state with the introducer system anchored in the tissue mass and the retraction of the VAB probe from the tissue mass.
Fig. 12 is a close-up view of area XII from Fig. 11 illustrating the pushrod extended from the first lumen to push the marker clip out of the introducer system and into the tissue mass.
Fig. 13 is a drawing similar to Fig. 12 illustrating the marker clip implanted in the tissue mass and the retraction of the introducer system from the tissue mass.
Figs. 14-16 are schematic illustrations showing a method of relocating the introducer system within the tissue mass.
Fig. 17 is a schematic illustration of an introducer system having distance markings on the first and second lumens.
Fig. 18 is an enlarged view of a second embodiment of the introducer system.
Fig. 19 is a sectional view of the introducer system taken along line 19-19 from Fig. 18.
Fig. 20 is an enlarged view of a third embodiment of the introducer system showing the anchor wire extended from the second lumen.
Fig. 21 is an enlarged view of the introducer system from Fig. 20 showing the anchor wire in a compressed configuration within the second lumen.
Fig. 22 is an enlarged view of the introducer system from Fig. 20 showing the anchor wire in a straight configuration within the second lumen.
Fig. 23 is an enlarged view of a fourth embodiment of the introducer system.
Fig. 24 is an enlarged view of a second embodiment of the VAB probe.
Fig. 25 is a drawing similar to Fig. 2 illustrating the introducer system contained within an outer cannula that is contained within the VAB probe.

### DESCRIPTION OF THE PREFERRED EMBODIMENT

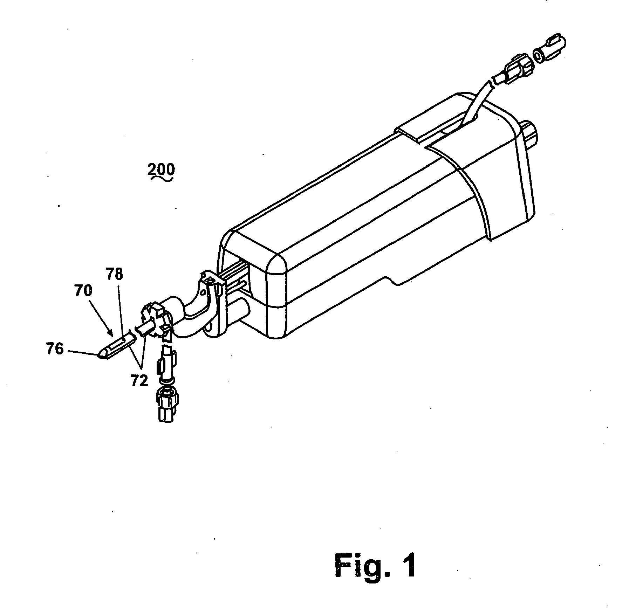

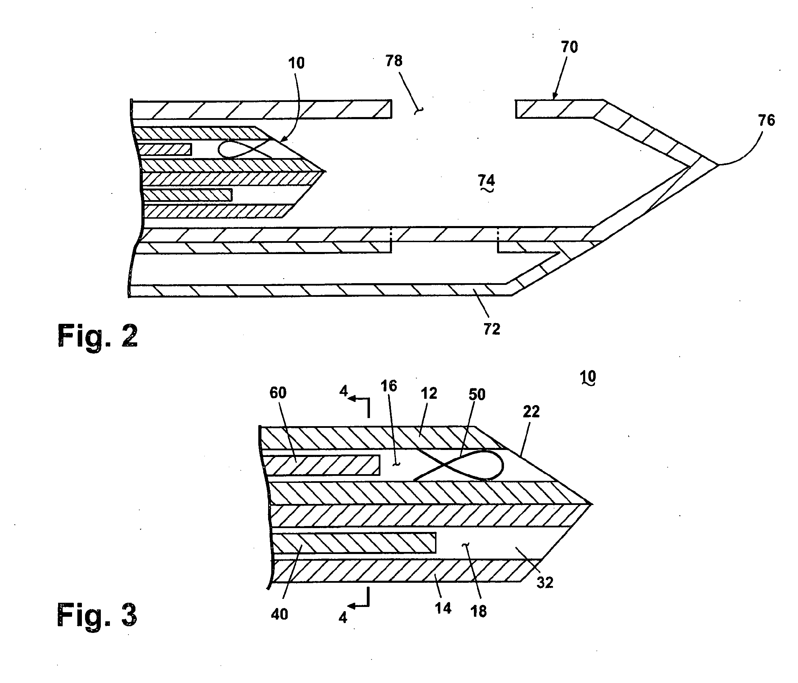

Referring now to the drawings and particularly to Fig. 2, an embodiment of the marker introducer system 10 is illustrated contained within a VAB probe 70 of a VAB system (Fig. 1). The VAB probe 70 comprises a vacuum chamber 72, a lumen 74, a closed insertion tip 76, and a proximal opening (not shown) into which the introducer system 10 can be inserted. An opening 78 in the cannula 72 allows a tissue sample to be taken from a tissue mass as previously described.

Referring additionally to Fig. 3, the introducer system 10 comprises a first sheath 12 and a second sheath 14 which respectively define a first lumen 16 and a second lumen 18. The first sheath 12 comprises an open distal insertion tip 22 and an open proximal end (not shown). The second sheath 14 comprises an open distal tip 32 and an open proximal end (not shown). An anchor wire 40 is contained within the second lumen 18 and a marker in the form of a clip 50 and a pushrod 60 are contained within the first lumen 16.

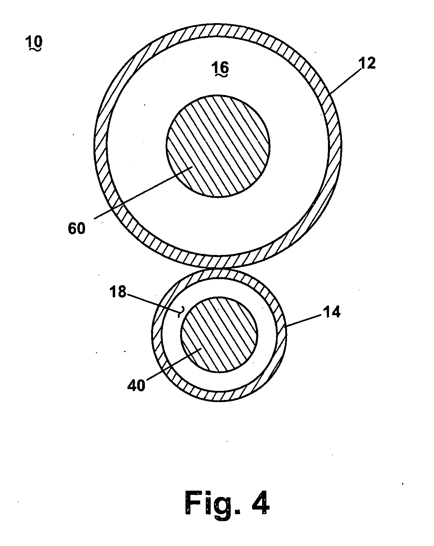

The sheaths 12, 14 are preferably independently fabricated from a biocompatible plastic that is flexible and bonded together as shown in Fig. 4. The gap between the sheaths 12, 14 and the corresponding pushrod 60 and anchor wire 40 is exaggerated in Fig. 3 to better discern the elements. One or both of the sheaths 12, 14 could also be formed from a coiled wire or any other biocompatible material that is sufficiently flexible such that the introducer system 10 can be inserted through the VAB probe and out of the opening 78.

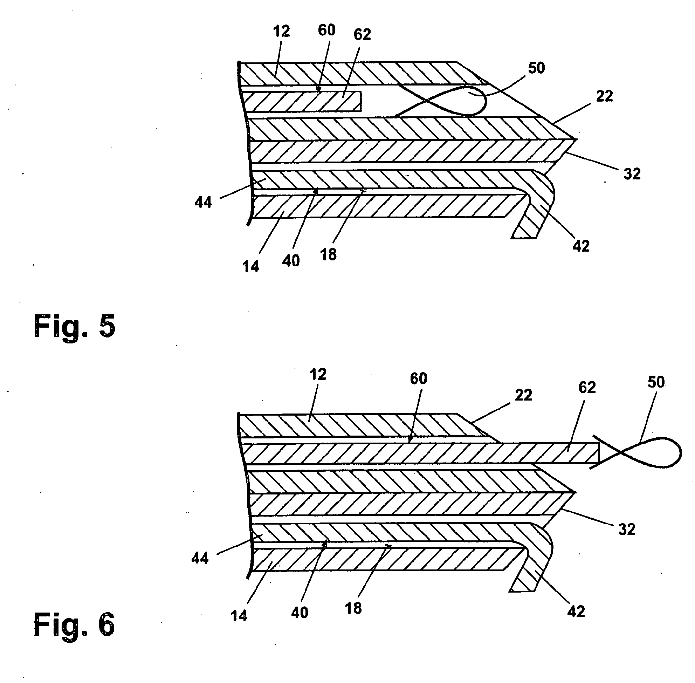

Referring to Fig. 5, the anchor wire 40 comprises a hook 42 and a thread 44. When mounted in the sheath 14 prior to implantation in the tissue mass, the hook 42 is contained within the second lumen 18 and a portion of the thread 44 extends exteriorly of from the proximal end of the sheath 14. The thread 44 is of sufficient length such that the proximal end of the thread 44 is exterior to the proximal end of the sheath 14 to manipulate the anchor wire 40 relative to the sheath 14.

The hook 42 is fabricated from a resilient, biocompatible material, for example a shape-memory alloy such as Nitinol. This allows the hook to assume a straight first configuration in the lumen as illustrated in Fig. 3, and a curved second configuration outside the lumen as illustrated in Fig. 5.

The hook 42 is preferably formed from the same wire as the thread 44 such that the hook is a continuation of the thread with the end of the hook 42 being connected to the thread 42 to complete hook 42. Alternately, the hook 42 and the thread 44 can be formed from different wires and or different materials. In either case, the hook 42 can be bonded or welded to the thread 44 to form the connection.

While the anchor wire 40 is shown having a hook 42 that engages the tissue mass, the anchor wire 40 can be formed with any one of a number of different anchors. For instance, as disclosed in U.S. Patent Application 2006/0111629, the anchor wire 40 can be formed with a diamond or square shaped anchor, a triangular shaped anchor, a circular shaped anchor, or any other anchor shape or type that provides a secure implantation of the introducer system 10 in the tissue mass. The shape of the anchor can be selected upon, for example, the density of the tissue into which the wire is to be placed, the size of the lesion, and/or the anchoring force required to implant the introducer system 10 in the tissue mass.

While only one anchor wire 40 is illustrated in the embodiments shown herein, it is understood that the introducer system 10 can comprise more than one anchoring device. For example, the introducer system 10 can have multiple anchor wires 40 loaded in the second sheath 14, or the introducer system 10 can have multiple sheaths that each hold one anchor wire 40. In either case, the anchor wires 40 can be configured to engage the tissue mass at different angles to provide for a more secure implantation of the introducer system 10.

Referring to Fig. 6, the pushrod 60 comprises a distal end 62 and a proximal end (not shown). The distal end 62 is used to force the clip 50 out of the sheath 12 and into the tissue mass. The pushrod 60 is of sufficient length such that the proximal end of the pushrod is exterior to the proximal end of the sheath 12 to manipulate the pushrod 60 relative to the sheath 12. The pushrod 60 can be made of any material that is sufficiently flexible in order to be threaded through the sheath 12, yet stiff enough to push the clip 50 out of the open tip 22 of the sheath 12.

The clip 50 can be any suitable type of marker that can be detected and located. The clip 50 can be imaged by an imaging technique or palpable through the skin and tissue. Types of imagable markers include markers that are echogenic, radiopaque, or a combination of these types. The imaging technique used locate the clip 50 can be a standard imaging system such as ultrasonography, mammography or magnetic resonance imaging.

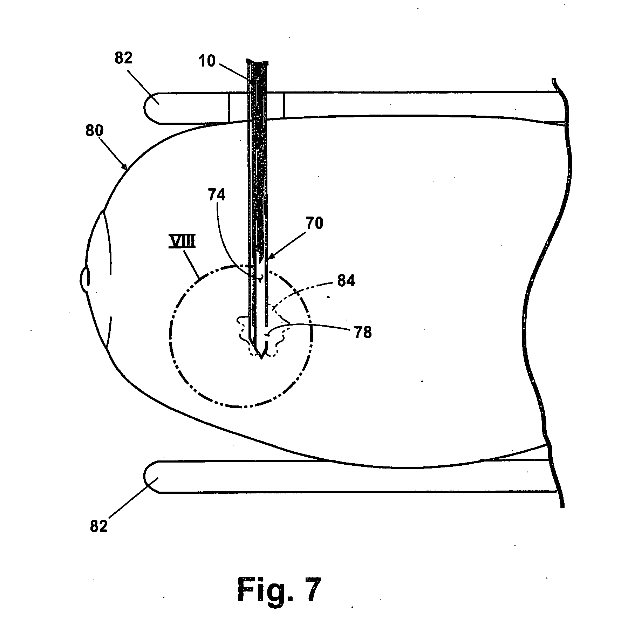

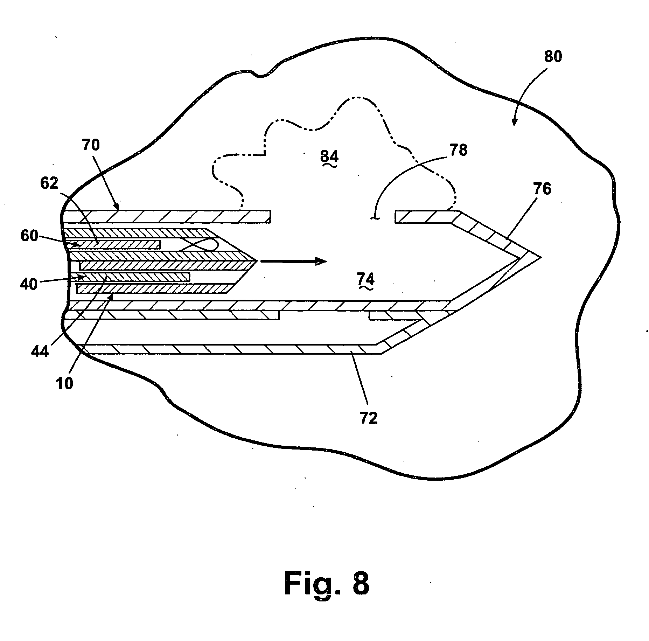

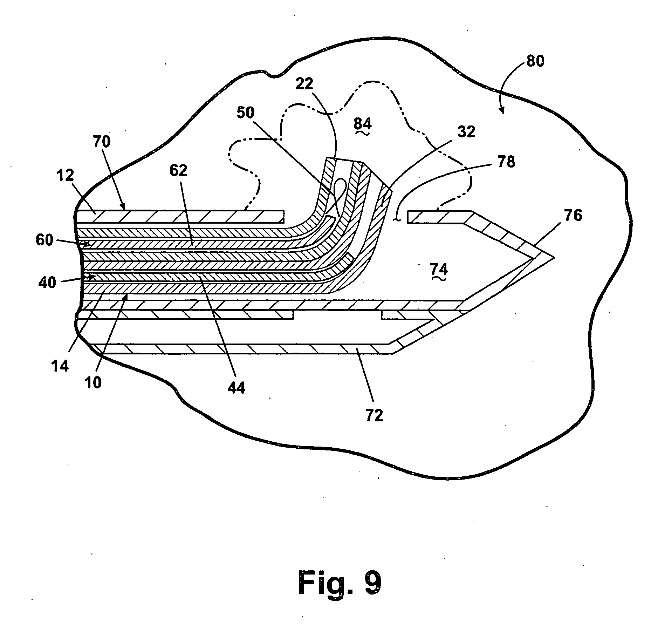

Referring to Figs. 7-13, the clip 50 is deployed into the tissue mass as follows. The VAB probe 70 is inserted into the tissue mass 80 illustrated as a breast that is compressed between two plates 82 and containing a target site 84. The target site can comprise a lesion or biopsy site. Referring to Fig. 8, after a VAB procedure has been performed during which a sample of tissue is taken from the target site 84, the introducer system 10 is inserted through the open proximal end of the VAB probe 70. Referring to Fig. 9, the introducer system is threaded through lumen 74 and through opening 78 so that the distal tips 22, 32 of the sheaths 12, 14 protrude into the tissue mass 80.

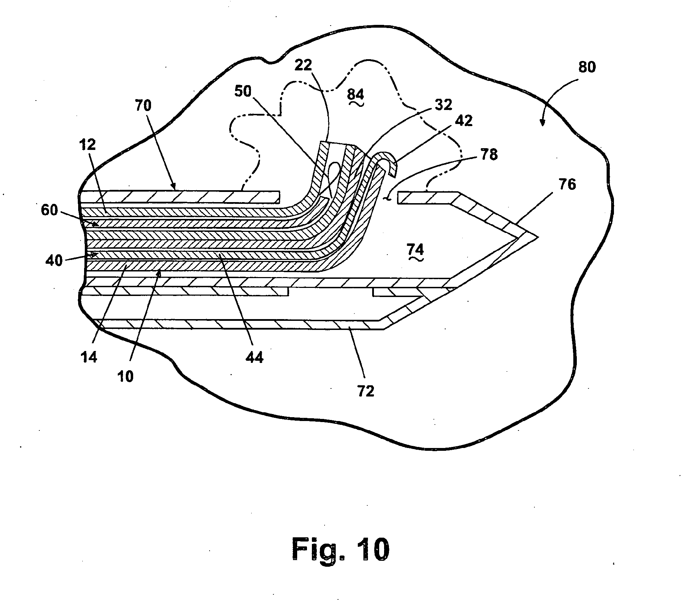

The introducer system 10 is then secured in the tissue mass 80 using the anchor wire 40. Referring to Fig. 10, the anchor wire 40 is embedded at the target site 84 by moving the thread 44 through lumen 18 relative to the sheath 14 such that the anchor wire 40 emerges from tip 32. As anchor wire 40 emerges from tip 32, hook 42 expands from the straightened first configuration to the curved second configuration. As it expands into the surrounding tissue, the hook 42 pierces the adjacent tissue to imbed the anchor wire 40 at the target site 84.

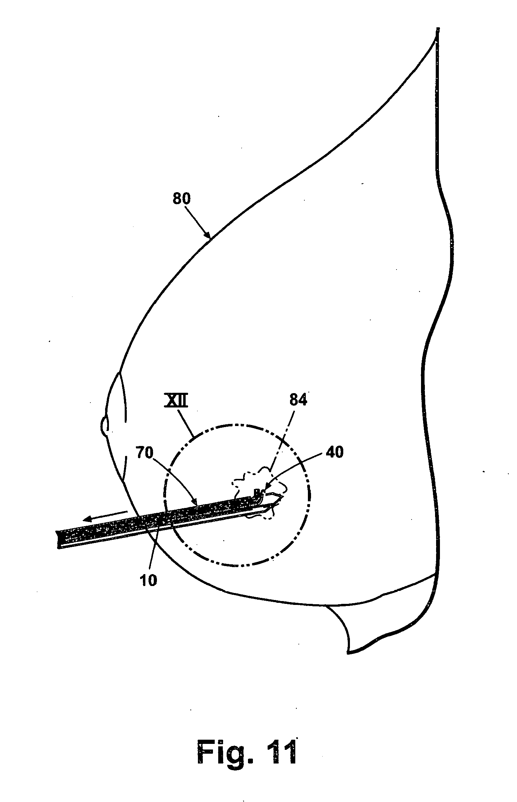

After anchoring the introducer system 10, the tissue mass 80 is uncompressed by removing the compression plates 82. The VAB probe is next retracted from the tissue mass 80 as illustrated by an arrow in Fig. 11. An image is taken of the tissue mass 80 to determine if the introducer system 10 has been correctly positioned at the target site 84. Correct positioning of the introducer system constitutes a placement that allows the clip 50 to be deployed at the target site 84 and thus is determined by the position of tip 22.

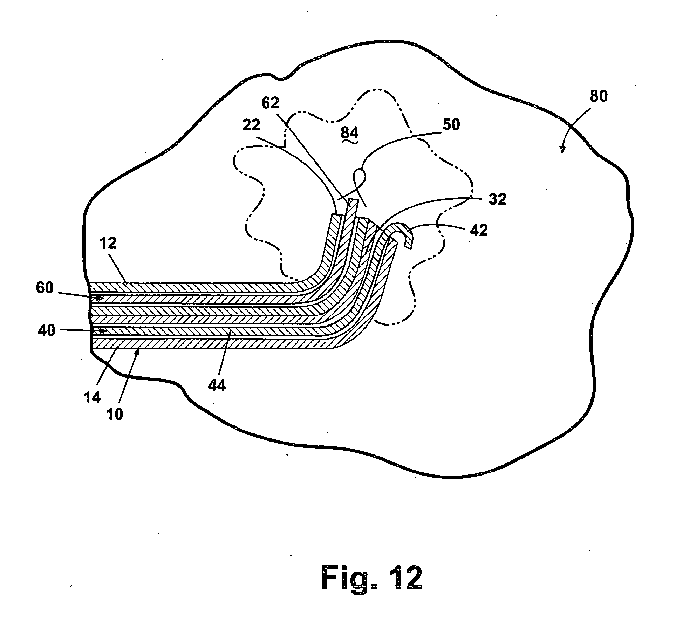

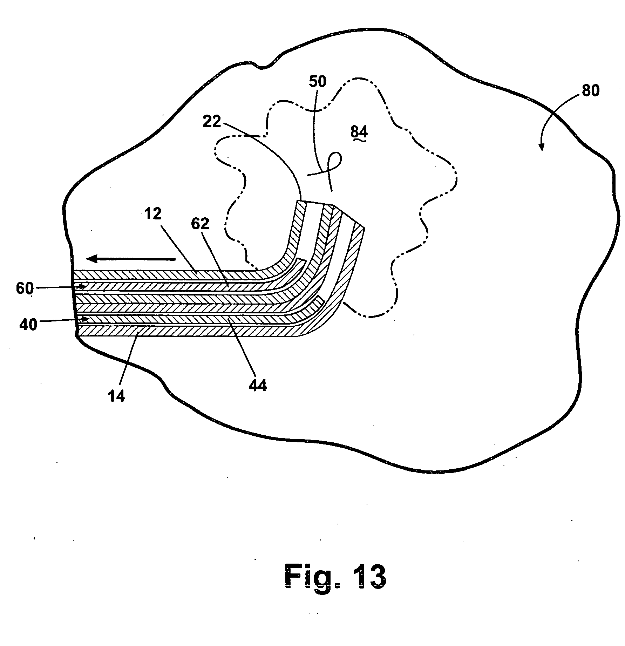

If the introducer system 10 is correctly positioned, the clip 50 is implanted in the tissue mass 80 to mark the target site 84. Referring to Fig. 12, the pushrod 60 is moved through lumen 16 relative to the sheath 12 such that the distal end 62 pushing the clip 50 emerges from tip 22 thus deploying clip 50 at the target site 84. The pushrod 60 and the anchor wire 40 are then retracted back into their respective sheaths 12 and 14, and the introducer system 10 is retracted from the tissue mass 80, leaving the clip 50 implanted at the target site 84 as illustrated in Fig. 13.

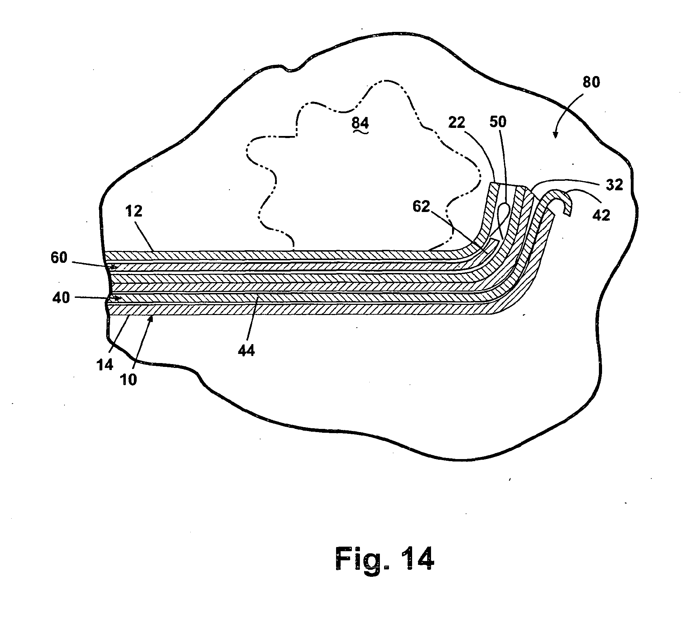

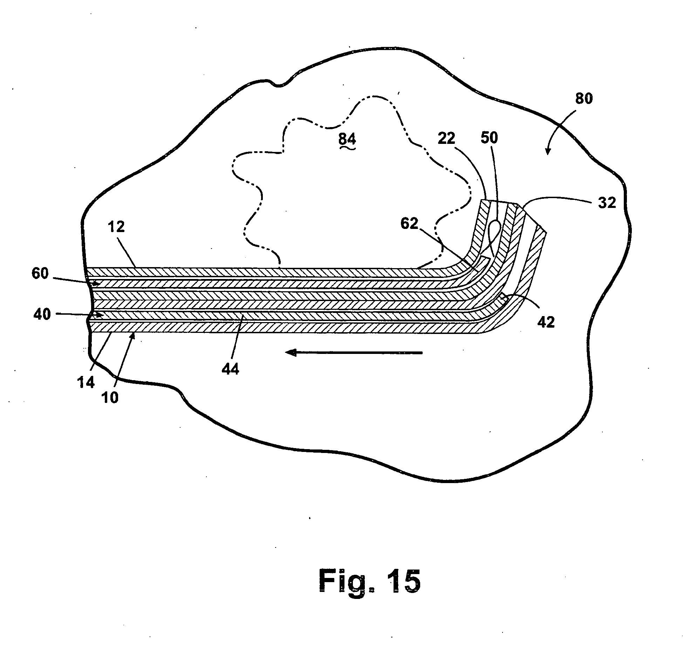

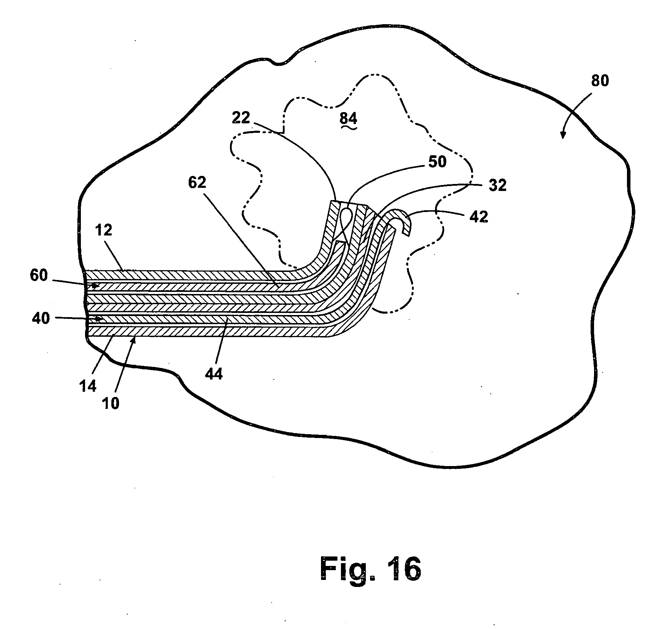

If it is determined by the image taken after the tissue mass 80 is uncompressed that the introducer system 10 has be incorrectly placed, the introducer system 10 can be repositioned within the tissue mass 80 as shown in Figs. 14-16. Repositioning is normally accomplished with the aid of an ultrasound. In the event of misplacement, it is most often the case that the introducer system 10 is deep to or beyond the target site 84 as illustrated in Fig. 14. Referring to Fig. 15, to reposition the introducer system, the anchor wire 40 is pulled back into lumen 18 by moving the thread 44 relative to the sheath 14. The introducer system 10 is next retracted back an appropriate distance such that the tips 22, 32 are at the target site 84. The introducer system is then secured in the tissue mass 80 using the anchor wire 40 and another image can be taken to confirm that the introducer system 10 is correctly positioned at the target site 84. The introducer system 10 can be repositioned as many times as necessary until the introducer system 10 is correctly positioned as illustrated in Fig. 16. The clip 50 is then implanted in the tissue mass 80 to mark the target site 84 as previously described.

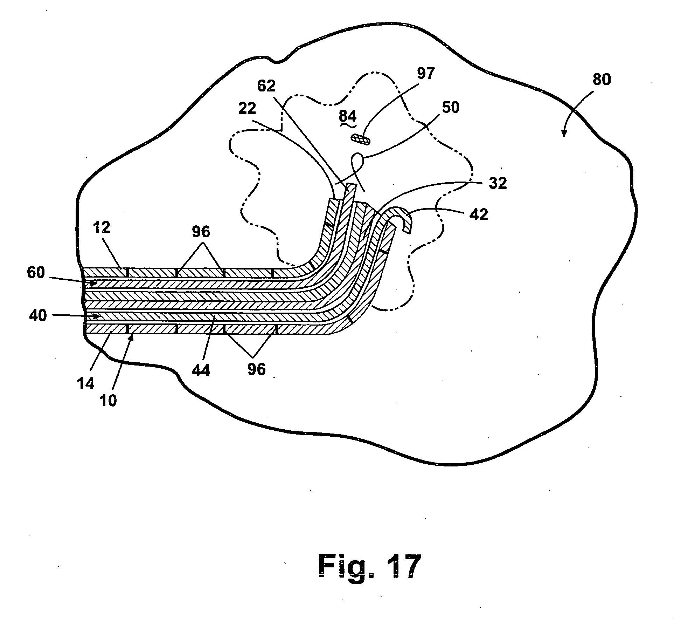

Referring to Fig. 17, to facilitate the repositioning of the introducer system 10, the sheaths 12, 14 could be provided with distance markings 96, for example centimeter markings that would enable the introducer system to be moved a distance determined from the image taken after the breast is uncompressed. Distance markings on the sheaths 12, 14 allow the introducer system 10 to be repositioned more accurately and reduces the possibility that the introducer system 10 has to be repositioned more than once to achieve correct placement of the introducer system 10.

Although a rare occurrence, the introducer system 10 can be misplaced shallow to or before the target site. To reposition the introducer system 10 in this case, a hollow cannula can be inserted over the introducer system 10 and then the cannula and introducer system 10 are advanced an appropriate distance to the target site 84. The cannula is next removed and the clip 50 is deployed.

FIG. 17 also illustrates the optional placement of a hemostatic agent 97 in addition to the placement of the clip 50. The hemostatic agent 97 can comprise a solid hemostatic agent such as a plug of collagen, chitosan, thrombin, Factor Xa, fibrinogen, nonsoluble polysaccharide, cellulose and dried gelatin; or a hemostatic agent in liquid form that is coated or impregnated in a bioabsorbable material. The hemostatic agent 97 can be loaded into the first sheath 12 along with the clip 50 and can be positioned relative to the clip 50 to be expelled prior to or just after the clip 50 as the push rod 60 is advanced. In another contemplated embodiment, the clip 50 can be coated with or encompassed by the hemostatic agent 97. The presence of the hemostatic agent 97 can prevent the clip 50 from being displaced due to bleeding at the target site 84.

While the VAB probe 70 is illustrated as the structure for providing a passageway into the tissue mass for the insertion of the introducer system, it should be noted that other insertion devices can be used and the introducer system is not limited to the VAB probe 70. For example, another insertion device can be a cannula with an axial opening or an opening in the side wall.

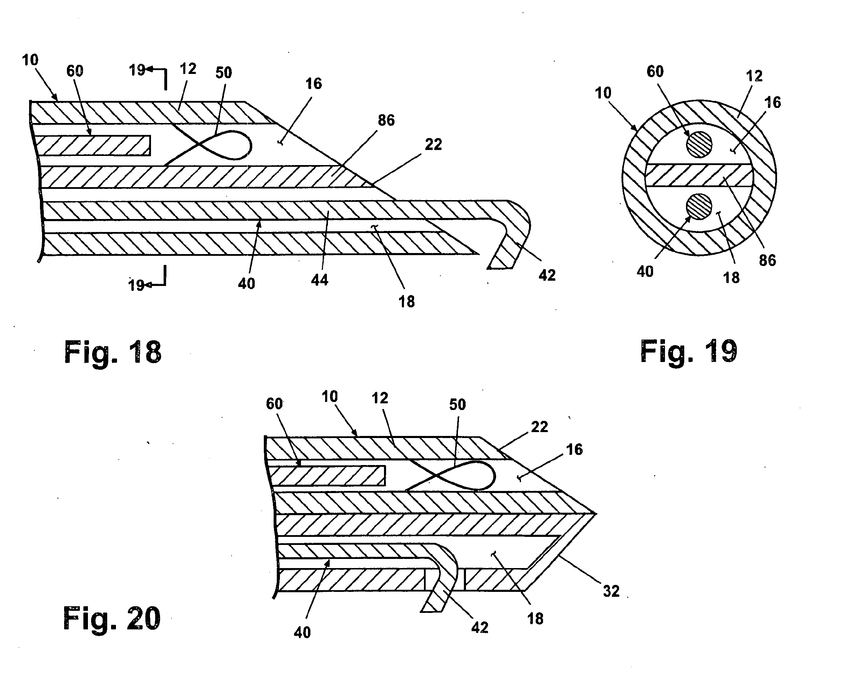

A second embodiment of the introducer system is shown in Figs. 18 and 19 where like elements are identified with the same reference numerals. In this embodiment, the first sheath 12 has a partition 86 that extends the length of the sheath and divides the sheath 12 into first lumen 16 and second lumen 18. Such a configuration has a smaller cross-sectional size as illustrated in Fig. 19 and the clip 50 is deployed in the same manner as described for the first embodiment of the introducer system 10.

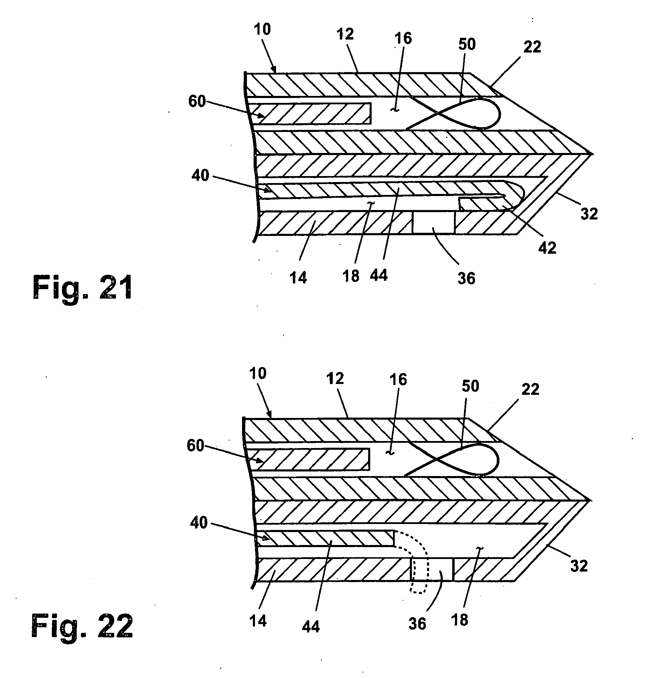

A third embodiment of the introducer system is shown in Figs. 20-22 where like elements are identified with the same reference numerals. In this embodiment, the tip 32 of the sheath 14 is closed and an opening 36 is provided in a side wall of the sheath 14, near the distal end of the sheath 14. As the anchor wire 40 is inserted into the sheath 14 it assumes the straight first configuration as shown in previous illustrations. When the hook 42 reaches the opening 36, it will assume the curved second configuration as it protrudes from the lumen 18 into the tissue mass to anchor the introducer system 10. Referring to Fig. 21, if it is then necessary to reposition the introducer system 10, the thread 44 is pushed forward, forcing the hook 42 through opening 36 and against the closed tip 32. Because of the forwardly-directed force on the wire 40, the hook 42 remains in the curved second position but is slightly compressed. After repositioning the introducer system 10, the thread 44 is pulled back, and the hook 42 exits the opening 36 to anchor the system into the tissue mass. Referring to Fig. 22, when the introducer system 10 is removed, the thread 44 is pulled back farther such that the hook 42 abuts the proximal edge of the opening 36 and assumes the straight first configuration as the hook 42 enter the second lumen 18.

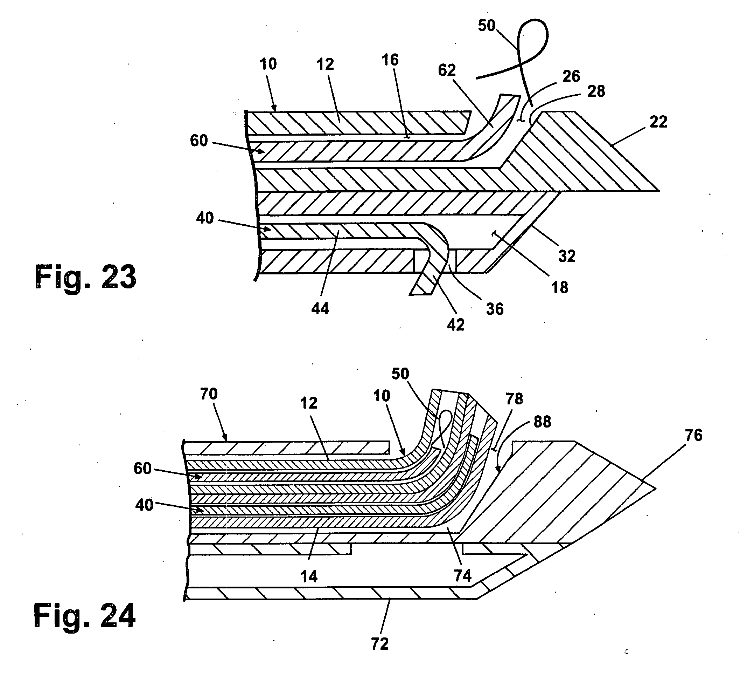

A fourth embodiment of the introducer system is shown in Fig. 23 where like elements are identified with the same reference numerals. In this embodiment, both tips 22, 32 are closed and openings 26, 36 are provided near the proximal end 24, 34 of the sheaths 12, 14, respectively. A ramp 28 is provided on the distal side of the opening 26 that occludes lumen 16 and prevents advancement of the clip 50 and the pushrod 60 beyond opening 26. The ramp 28 is angled to guide the clip 50 and the pushrod 60 upward and through the opening 26.

The VAB probe 70 can be altered in a similar fashion to facilitate the movement of the introducer system 10 out of the probe 70. A second embodiment of the probe 70, shown in FIG. 24 where like elements are identified with the same reference numerals, has a ramp 88 formed on the distal side of the opening 78 such that it occludes lumen 74 and prevents the introducer system 10 from advancing beyond the opening 78. The ramp 80 is angled to guide the introducer system 10 upwards and through opening 78. While the second embodiment of probe 70 is shown in conjunction with the first embodiment of the introducer system 10, it is understood that any embodiment of the introducer system 10 can be used with the second embodiment of the probe 70.

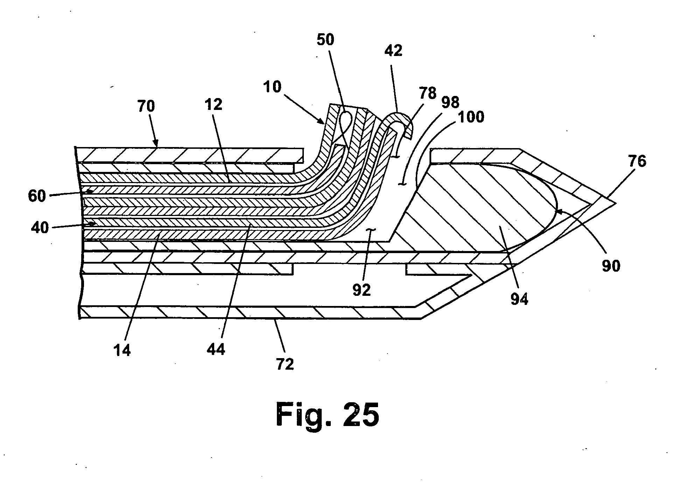

Referring to FIG. 25, the introducer system 10 can also be inserted through an outer cannula 90 to facilitate the movement of the introducer system 10 out of the probe 70. The cannula 90 defines a lumen 92 and comprises a closed distal end 94 and a proximal end (not shown). An opening 98 near the distal end 94 is provided with a ramp 100. The opening 98 is located on the cannula 90 such that when the cannula 90 is fully inserted, the opening 98 is aligned with opening 78. The cannula 90 is considered to be fully inserted into the probe 70 when the closed end 94 contacts the closed insertion tip 76, thus aligning opening 98 with opening 78. The cannula 90 has an outer diameter sized so that is can easily fit through the lumen 74 of the probe 70 and an inner diameter sized so that the introducer system 10 can easily fit through lumen 92.

To deploy the clip 50, the outer cannula 90 is first inserted into the probe 70 and pushed forward until it is fully inserted. Full insertion of the cannula 90 can be determined when resistance is felt against the further forward movement of the cannula 90. Then, the introducer system 10 is inserted into the cannula 90 such that the introducer system 10 is guided up the ramp 100 and out of the opening 98. Next, the introducer system 10 is anchored by the anchor wire 40 and the probe 70 and cannula 90 are simultaneously retracted leaving the introducer system 10 in the tissue mass. The clip 50 is then deployed following the same steps as previously described.

While the invention has been specifically described in connection with certain specific embodiments thereof, it is to be understood that this is by way of illustration and not of limitation.

## Claims

1. An apparatus for implanting a locatable marker at a target site within a tissue mass comprising:
an insertion device (70) comprising a lumen (74) having an exit opening;
an introducer system (10) comprising a sheath (12) slidably received within the insertion device lumen and comprising a first lumen (16) having a distal opening; and
a locatable marker (50) received within the first lumen and deployable through the distal opening;
wherein the insertion device can be located within the tissue mass,
**characterized in that** the apparatus further comprises an anchor (40) operably coupled to the introducer system (10) to fix the location of the introducer system in the tissue mass; and wherein the introducer system can be inserted into the tissue mass through the exit opening of the insertion device, and the anchor (40) can fix the position of the introducer system in the tissue mass for deployment of the locatable marker (50) at the target site.

2. The apparatus according to claim 1 wherein the introducer system (10) comprises a second lumen (18) having a distal opening, with the anchor (40) received within the second lumen and deployable through the distal opening.

3. The apparatus according to claim 1 or 2 wherein the introducer system (10) comprises a distal terminal end (22), and the distal terminal end comprises an insertion tip.

4. The apparatus according to any of claims 1-3 wherein at least one of the introducer system (10) distal openings are formed in the terminal end (22) of the introducer system (10).

5. The apparatus according to claim any of claims 1-4 wherein at least one (26; 36) of the introducer system (10) distal openings are formed in a side wall of the introducer system (10).

6. The apparatus according to claim 5 wherein the at least one (26) of the introducer system distal openings formed in the side wall comprises a ramp (28) to guide the locatable marker (50) through the at least one of the introducer system distal openings formed in the side wall.

7. The apparatus according to any of claims 1-6 and further comprising a pushrod (60) slidably received within the first lumen that deploys the locatable marker through the distal opening.

8. The apparatus according to any of claims 1-7 wherein the anchor (40) comprises an anchor wire.

9. The apparatus according to claim 8 wherein the anchor wire (40) is operable between a straight configuration where the anchor wire is contained within the second lumen and a curved configuration where the anchor wire is extended through the distal opening.

10. The apparatus according to claim 9 wherein the anchor wire (40) is embedded in the tissue mass in the curved configuration.

11. The apparatus according to any of claims 1-10 and further comprising a cannula (90) received within the insertion device lumen (74), the cannula comprising a lumen (92) having a distal opening, with the introducer system (10) received within the cannula lumen (92).

12. The apparatus according to claim 11 wherein the cannula distal opening comprises a ramp (100) to guide the introducer system (10) through the cannula distal opening.

13. The apparatus according to any of claims 1-12 wherein the insertion device is a biopsy probe.

14. The apparatus according to claims 13 wherein the probe is a vacuum-assisted biopsy probe.

15. The apparatus according to any of claims 1-14 wherein the exit opening comprises a ramp (88).

16. The apparatus according to any of claims 1-15 wherein the introducer system (10) is flexible.

17. The apparatus according to any of claims 1-16 wherein the introducer system (10) comprises distance markings.

18. The apparatus according to any of claims 1-17 and further comprising a pair of compression plates (82) for compressing the tissue mass prior to location of the insertion device into the tissue mass at the target site and for decompressing the tissue mass prior to implantation of the locatable marker.

19. The apparatus according to any of claims 1-18 and further comprising a hemostatic agent (97) received within the first lumen and deployable through the distal opening.

20. The apparatus according to any of claims 1-19 wherein the locatable marker (50) is one of an imaging marker and a palpable marker.

21. The apparatus according to any of claims 1-20 wherein the locatable marker (50) is a clip.

## Patentansprüche

1. Gerät zur Implantation eines lokalisierbaren Markers an einer Zielstelle in einer Gewebemasse, das Folgendes umfasst: eine Einführungsvorrichtung (70) mit einem Lumen (74) mit einer Austrittsöffnung; ein Einführungssystem (10) mit einer Schleuse (12), die gleitend im Lumen der Einführungsvorrichtung aufgenommen ist, und mit einem ersten Lumen (16) mit einer distalen Öffnung; und einen lokalisierbaren Marker (50), der im ersten Lumen aufgenommen ist und durch die distale Öffnung eingesetzt werden kann; wobei die Einführungsvorrichtung in der Gewebemasse lokalisiert sein kann, **dadurch gekennzeichnet, dass** das Gerät ferner einen Anker (40) umfasst, der mit dem Einführungssystem (10) in Wirkverbindung steht, um die Lokalisation des Einführungssystems in der Gewebemasse zu fixieren; und wobei das Einführungssystem durch die Austrittsöffnung der Einführungsvorrichtung in die Gewebemasse eingeführt werden kann, und wobei der Anker (40) zum Einsetzen des lokalisierbaren Markers (50) an der Zielstelle die Position des Einführungssystems in der Gewebemasse fixieren kann.

2. Gerät nach Anspruch 1, wobei das Einführungssystem (10) ein zweites Lumen (18) mit einer distalen Öffnung umfasst, wobei der Anker (40) im zweiten Lumen aufgenommen ist und durch die distale Öffnung eingesetzt werden kann.

3. Gerät nach Anspruch 1 oder 2, wobei das Einführungssystem (10) ein distales terminales Ende (22) umfasst und das distale terminale Ende eine Einführungsspitze umfasst.

4. Gerät nach einem der Ansprüche 1-3, wobei zumindest eine der distalen Öffnungen des Einführungssystems (10) im terminalen Ende (22) des Einführungssystems (10) geformt ist.

5. Gerät nach einem der Ansprüche 1-4, wobei zumindest eine (26; 36) der distalen Öffnungen des Einführungssystems (10) in einer Seitenwand des Einführungssystems (10) geformt ist.

6. Gerät nach Anspruch 5, wobei die zumindest eine distale Öffnung (26) des Einführungssystems in der Seitenwand eine Rampe (28) zur Führung des lokalisierbaren Markers (50) durch die zumindest eine distale Öffnung des Einführungssystems in der Seitenwand umfasst.

7. Gerät nach einem der Ansprüche 1-6, das ferner eine Schubstange (60) umfasst, die gleitend im ersten Lumen aufgenommen ist und den lokalisierbaren Marker durch die distale Öffnung einsetzt.

8. Gerät nach einem der Ansprüche 1-7, wobei der Anker (40) einen Verankerungsdraht umfasst.

9. Gerät nach Anspruch 8, wobei der Verankerungsdraht (40) zwischen einer geraden Konfiguration, in welcher der Verankerungsdraht im zweiten Lumen eingeschlossen ist, und einer gebogenen Konfiguration, in welcher der Verankerungsdraht durch die distale Öffnung ausgestreckt ist, betätigt werden kann.

10. Gerät nach Anspruch 9, wobei der Verankerungsdraht (40) in der gebogenen Konfiguration in der Gewebemasse eingebettet ist.

11. Gerät nach einem der Ansprüche 1-10, das ferner eine Kanüle (90) umfasst, die im Lumen (74) der Einführungsvorrichtung aufgenommen ist, wobei die Kanüle ein Lumen (92) mit einer distalen Öffnung umfasst und wobei das Einführungssystem (10) im Kanülenlumen (92) aufgenommen ist.

12. Gerät nach Anspruch 11, wobei die distale Öffnung der Kanüle eine Rampe (100) zur Führung des Einführungssystems (10) durch die distale Öffnung der Kanüle umfasst.

13. Gerät nach einem der Ansprüche 1-12, wobei die Einführungsvorrichtung eine Biopsiesonde ist.

14. Gerät nach Anspruch 13, wobei die Sonde eine vakuumgestützte Biopsiesonde ist.

15. Gerät nach einem der Ansprüche 1-14, wobei die Austrittsöffnung eine Rampe (88) umfasst.

16. Gerät nach einem der Ansprüche 1-15, wobei das Einführungssystem (10) flexibel ist.

17. Gerät nach einem der Ansprüche 1-16, wobei das Einführungssystem (10) Abstandsmarkierungen umfasst.

18. Gerät nach einem der Ansprüche 1-17, das ferner ein Paar Kompressionsplatten (82) zur Kompression der Gewebemasse vor der Lokalisation der Einführungsvorrichtung in der Gewebemasse an der Zielstelle und zur Dekompression der Gewebemasse vor Implantation des lokalisierbaren Markers umfasst.

19. Gerät nach einem der Ansprüche 1-18, das ferner ein hämostatisches Mittel (97) umfasst, das im ersten Lumen aufgenommen ist und durch die distale Öffnung eingesetzt werden kann.

20. Gerät nach einem der Ansprüche 1-19, wobei der lokalisierbare Marker (50) ein bildgebender Marker oder ein tastbarer Marker ist.

21. Gerät nach einem der Ansprüche 1-20, wobei der lokalisierbare Marker (50) ein Clip ist.

## Revendications

1. Dispositif pour implanter un marqueur localisable à un site cible dans une masse tissulaire comportant :
un dispositif d'insertion (70) comportant une lumière (74) présentant une ouverture de sortie ;
un système introducteur (10) comportant une gaine (12) reçue de manière coulissante dans la lumière du dispositif d'insertion et comportant une première lumière (16) présentant une ouverture distale ;
et
un marqueur localisable (50) reçu dans la première lumière et déployable à travers l'ouverture distale ;
dans lequel le dispositif d'insertion peut être localisé dans la masse tissulaire, **caractérisé en ce que** le dispositif comporte en outre une ancre (40) reliée de manière fonctionnelle au système introducteur (10) pour maintenir en place le système introducteur dans la masse tissulaire ; et dans lequel le système introducteur peut être inséré dans la masse tissulaire par l'ouverture de sortie du dispositif d'insertion, et l'ancre (40) peut fixer la position du système introducteur dans la masse tissulaire pour le déploiement du marqueur localisable (50) au site cible.

2. Dispositif selon la revendication 1 dans lequel le système introducteur (10) comporte une seconde lumière (18) présentant une ouverture distale, avec l'ancre (40) reçue dans la seconde lumière et déployable à travers l'ouverture distale.

3. Dispositif selon la revendication 1 ou 2 dans lequel le système introducteur (10) comporte une extrémité terminale distale (22), et l'extrémité terminale distale comporte un bout d'insertion.

4. Dispositif selon l'une quelconque des revendications 1 à 3 dans lequel au moins une desdites ouvertures distales du système introducteur (10) est formée dans l'extrémité terminale (22) du système introducteur (10).

5. Dispositif selon l'une quelconque des revendications 1 à 4 dans lequel au moins une (26 ; 36) des ouvertures distales du système introducteur (10) est formée dans une paroi latérale du système introducteur (10).

6. Dispositif selon la revendication 5 dans lequel ladite au moins une (26) ouverture distale du système introducteur formée dans la paroi latérale comporte une rampe (28) pour guider le marqueur localisable (50) dans ladite au moins une ouverture distale du système introducteur formée dans la paroi latérale.

7. Dispositif selon l'une quelconque des revendications 1 à 6 et comportant en outre une tige-poussoir (60) reçue de manière coulissante dans la première lumière qui déploie le marqueur localisable à travers l'ouverture distale.

8. Dispositif selon l'une quelconque des revendications 1 à 7 dans lequel l'ancre (40) comporte un fil d'ancrage métallique.

9. Dispositif selon la revendication 8 dans lequel le fil d'ancrage métallique (40) fonctionne entre une configuration droite dans laquelle le fil métallique est contenu dans la seconde lumière et une configuration courbe dans laquelle le fil d'ancrage métallique s'étend dans l'ouverture distale.

10. Dispositif selon la revendication 9 dans lequel le fil d'ancrage métallique (40) est noyé dans la masse tissulaire dans la configuration courbe.

11. Dispositif selon l'une quelconque des revendications 1 à 10 et comportant en outre une canule (90) reçue dans la lumière du dispositif d'insertion (74), la canule comportant une lumière (92) présentant une ouverture distale, avec le système introducteur (10) reçu dans la lumière de canule (92).

12. Appareil selon la revendication 11 dans lequel l'ouverture distale de la canule comporte une rampe (100) pour guider le système introducteur (10) à travers l'ouverture distale de la canule.

13. Dispositif selon l'une quelconque des revendications 1 à 12 dans lequel le dispositif d'insertion est une sonde de biopsie.

14. Dispositif selon la revendication 13 dans lequel la sonde est une sonde de biopsie à aspiration assistée par dépression.

15. Dispositif selon l'une quelconque des revendications 1 à 14 dans lequel l'ouverture de sortie comporte une rampe (88).

16. Dispositif selon l'une quelconque des revendications 1 à 15 dans lequel le système introducteur (10) est souple.

17. Dispositif selon l'une quelconque des revendications 1 à 16 dans lequel le système introducteur (10) comporte des marques de distance.

18. Dispositif selon l'une quelconque des revendications 1 à 17 et comportant en outre une paire de plaques de compression (82) pour comprimer la masse tissulaire avant la localisation du dispositif d'insertion dans la masse tissulaire au site cible et pour décomprimer la masse tissulaire avant l'implantation du marqueur localisable.

19. Dispositif selon l'une quelconque des revendications 1 à 18 et comportant en outre un agent hémostatique (97) reçu dans la première lumière et déployable à travers l'ouverture distale.

20. Dispositif selon l'une quelconque des revendications 1 à 19 dans lequel le marqueur localisable (50) est un marqueur d'imagerie et/ou un marqueur palpable.

21. Dispositif selon l'une quelconque des revendications 1 à 20 dans lequel le marqueur localisable (50) est une pince.
